# EUROPEAN PATENT APPLICATION

(11) **EP 1 683 479 A1**
(43) Date of publication of application: **26.07.2006**
(21) Application number: 06100362.0
(22) Date of filing: 16.01.2006
(51) Int. Cl.: A61B 5/00, A61B 5/0404, A61B 5/024

(54) **System for monitoring an individual's physiological state, and a computer software product**

(30) Priority: 19.01.2005 FI 20055026
(71) Applicant: Polar Electro Oy, 90440 Kempele (FI)
(72) Inventor: HEIKKILÄ, Ilkka, 90240, OULU (FI)
(74) Representative: Antila, Harri Jukka Tapani

(57) **Abstract**

A system comprising: a user-specific performance monitor (102,104) for generating physiological measurement data about the user, the physiological measurement data being used in the physiological state-monitoring program, the user-specific performance monitor comprising processing means for executing an application, the application executing at least part of the physiological state-monitoring program by processing monitoring program data of the physiological state-monitoring program; a mobile communications system terminal (106) configured to execute said application; a personal computer (110) configured to execute said application; and update means for updating the user-specific performance monitor, the mobile communications system terminal and the personal computer with updateable monitoring program data by using wireless data transfer.

## Description

### FIELD

The invention relates to a system for monitoring an individual's physiological state by means of a physiological state-monitoring program, to a personal performance monitor and to a computer software product.

### BACKGROUND

A method of monitoring the physiological state in intended to monitor and follow the development of an individual's physiological state, such as weight and/or physical performance, in time. Physiological target values, associated with for instance the balance between energy intake and energy consumption, are set in the method of monitoring the physiological state. The fulfilment of the target values is typically monitored by means of physiological measurement data generated from the person's electrocardiogram and the input given by the user.

An essential element in a physiological state-monitoring program is a user-specific performance monitor, which generates physiological measurement data about the user and records other measurands characteristic of the environmental conditions, for example. A user-specific heart rate monitor is a small-sized device that the user carries along and that has a limited usability implemented by a user interface, such as a display and a keyboard. Consequently, it is useful to develop means for decreasing the limitations set by the user interface of a user-specific performance monitor.

### BRIEF DESCRIPTION

The object of the invention is to implement a system, a personal performance monitor and a computer software product in a manner achieving easy usability of the physical state-monitoring program. As a first aspect of the invention there is provided a system for monitoring an individual's physiological state by means of a physiological state-monitoring program, comprising: a user-specific performance monitor to be connected wirelessly to the system for generating physiological measurement data about the user, the physiological measurement data being used in the physiological state-monitoring program, the user-specific performance monitor comprising processing means for executing an application, the application executing at least part of the physiological state-monitoring program by processing monitoring program data of the physiological state-monitoring program, the system further comprising: a mobile communications system terminal to be connected wirelessly to the system and configured to execute said application; a personal computer to be connected wirelessly to the system and configured to execute said application; and update means for updating the user-specific performance monitor, the mobile communications system terminal and the personal computer with updateable monitoring program data by using wireless data transfer.

As a second aspect of the invention there is provided a computer software product including coded instructions for executing a computer process in a system for monitoring an individual's physiological state by means of a physiological state-monitoring program, the system comprising a user-specific performance monitor to be connected wirelessly to the system for generating physiological measurement data about the user, the physiological measurement data being used in the physiological state-monitoring program, and the system further comprising a mobile communications system terminal to be connected wirelessly to the system and a personal computer to be connected wirelessly to the system, and the computer software program comprising: coded instructions for executing an application in a measuring unit, in the mobile communications system terminal and in the personal computer, the application executing at least part of the physiological state-monitoring program by processing the monitoring program data of the physiological state-monitoring program; and coded instructions for updating the user-specific performance monitor, the mobile communications system terminal and the personal computer with updateable monitoring program data by using wireless data transfer.

As another aspect of the invention there is provided a user-specific performance monitor for generating physiological measurement data about a user, the user-specific performance monitor being wirelessly connectable to a system, the system being used to monitor an individual's physiological state by means of a physiological state-monitoring program, the physiological state-monitoring program using physiological measurement data, the user-specific performance monitor comprising: processing means for executing an application, the application executing at least part of the physiological state-monitoring program by processing monitoring program data of the physiological state-monitoring program, the application further being executable in a mobile communications system terminal to be connected wirelessly to the system and in a personal computer to be connected wirelessly to the system; and update means for updating the system with updateable monitoring program data by using wireless data transfer.

Preferred embodiments of the invention are described in the dependent claims.

The invention is based on at least one of the applications of the physiological state-monitoring program being executable in any system element, and each system element being updated with updateable monitoring program data of the physiological state-monitoring program.

The method and system of the invention bring about a plurality of advantages. An advantage is that the use of a physiological state-monitoring program is facilitated in various operating situations. This is the result of the ability to use at least one application of the monitoring program in any system element.

### LIST OF FIGURES

In the following, the invention will be described in more detail in connection with preferred embodiments with reference to the accompanying drawings, in which
Figure 1 shows an example of the structure of the system;
Figure 2 shows an example of the structure of a user-specific performance monitor;
Figure 3 shows an example of the structure of a mobile communications system terminal, and
Figure 4 shows and example the structure of a personal computer.

### DESCRIPTION OF EMBODIMENTS

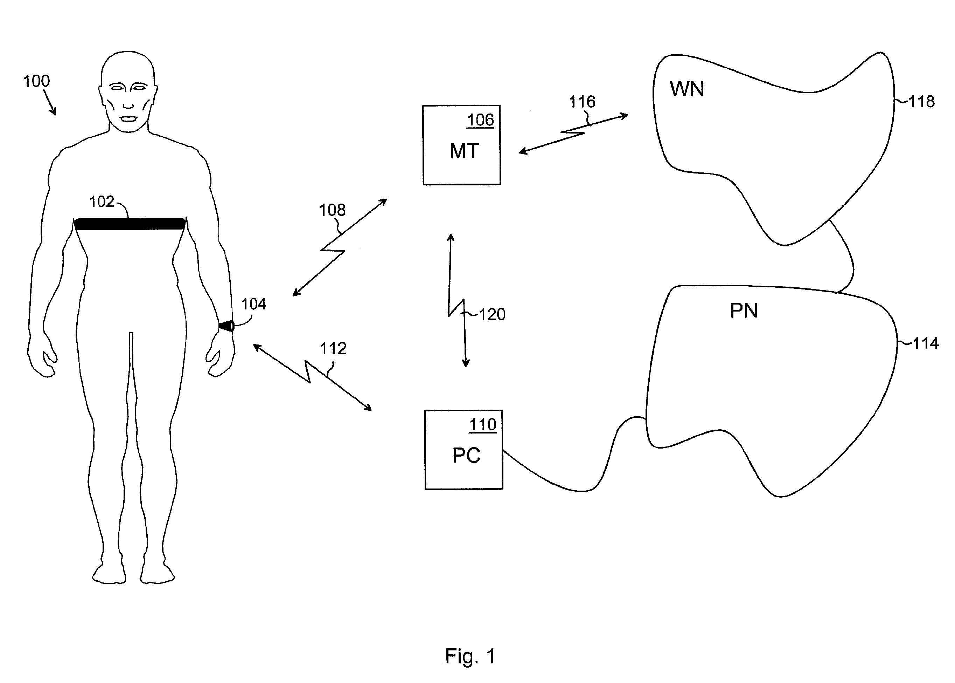

Figure 1 shows a system comprising a user-specific performance monitor 102, 104, a mobile communications system terminal 106, and a personal computer 110. The user-specific performance monitor 102, 104 may comprise a transmitter part 102 and a receiver part 104.

The user-specific performance monitor 102, 104 is characterized in that the user 100 who is the target of the performance measurement observes him/herself the performance monitor 102, 104, and issues the required operational commands to the performance monitor 102, 104. In this context, the user-specific performance monitor 102, 104 will be called briefly a performance monitor 102, 104.

The mobile communications system terminal 106 can be connected wirelessly via a radio interface 116 to a mobile communications system infrastructure 118, which, in turn, may be connected to a public data network 114, such as the Internet. The mobile communications system terminal 106 is also known as a mobile phone, a mobile, and user equipment. In this context, the mobile communications system terminal 106 will be called briefly a terminal 106.

The mobile communications system is GSM (Global System for Mobile Communications), UMTS (Universal Mobile Telecommunications System) or the CDMA2000 system, for example. However, the solution presented is not restricted to said mobile communications systems.

In an embodiment, the performance monitor 102, 104 may be in a wireless connection 108 to the terminal 106.

The personal computer 110 may be a portable or a fixedly mounted desk computer, which may be connected to the public data network 114. In this context, the personal computer 110 will be called briefly a computer 110.

In an embodiment, the performance monitor 102, 104 may be in a wireless connection 112 to the computer 110.

In an embodiment, the terminal 106 may be in a wireless connection 120, such as a Bluetooth connection and/or an infrared connection to the computer 110.

The system implements functions of the physiological state-monitoring program, by means of which the user 100 tends to monitor and follow the development of his/her physiological state, such as weight and/or physical performance, in time.

The operation of the physiological state program is typically based on processing monitoring program data. The monitoring program data may comprise for instance the following monitoring data elements:
- user data, such as the user's initial weight, length, sex and age
- the user's target weight
- the user's momentary weight
- the user's target energy intake
- the user's target energy consumption
- the user's energy intake
- the user's energy consumption
- form of physical activity
- physiological measurement data generated by the performance monitor from the electrocardiogram
- physiological measurement data obtained from the activity measurement of the performance monitor
- exercise journal entry
- target value for heart rate variable, such as rest heart rate or maximum heart rate
- limit value for heart rate variable
- limit value for variable descriptive of performance
- limit value for environmental parameter, such as temperature, pressure and height
- other variable input by the user, such as waistline and number of exercises

In an embodiment, the physiological state-monitoring program is a weight-monitoring program. This being so, the user 100 first inputs for instance his/her initial weight, target weight, and a time target into the weight monitoring program. During the weight-monitoring program, the user 100 inputs for instance the amount of energy obtained daily into the system. The weight monitoring program process determines the amount of energy consumed from physiological measurement data generated by the performance monitor 102, 104, and calculates the energy balance. The physiological measurement data can be generated from the user's 100 electrocardiogram or excitation information, whereby the physiological measurement data contain for instance heart rate variable values and/or parameters associated with energy consumption. The weight monitoring program may further observe the development of the energy balance daily and generate for instance new target values for energy intake and/or energy consumption for achievement of the weight target set. The observation may be performed for instance by the weight-monitoring program generating a program calendar wherein the performances and inputs occurred during the weight-monitoring program are recorded.

The physiological state-monitoring program is implemented by using applications that process the monitoring program data. At least one of the applications can be executed in the performance monitor 102, 104, in the terminal 106 and in the computer 110. This being so, the performance monitor 102, 104, the terminal 106 and the computer 110 are able to execute the same functionality of the physiological monitoring program data.

In the system, the performance monitor 102, 104, the terminal 106 and the computer 110 are updated with updateable monitoring program data. The update is performed by using wireless connections 108, 112, 120.

The need to update the monitoring program data may be the consequence of a change in the monitoring program data in the system. The monitoring program data may change as a result of the user's 100 action and/or a physiological state-monitoring program process, for example.

Updating the monitoring program data enables the use of an application of the physiological state-monitoring program from any system part 102, 104, 106, 110. This being so, the user 100 is able to select the most suitable user interface for using the physiological state-monitoring program.

In an embodiment, the application executes a monitoring program data input process. In this case, the monitoring program data can be input into the system from the performance monitor 102, 104, the mobile communications system terminal 106 and the computer 110. The monitoring program data may be generated by the user and/or a process of the physiological state-monitoring program. For example, the user 100 may input the amount of energy intake and/or a journal entry into the system by using the computer 110. This being so, the energy amount and/or the journal entry may be updated via the connection 120 to the terminal 106. Furthermore, the terminal 106 may update the performance monitor 102, 104 with the energy amount and/or journal entry. In an embodiment, the performance monitor 102, 104 is first updated with the energy amount and/or journal entry, after which the performance monitor 102, 104 updates the terminal 106 with the energy amount and/or journal entry.

The update order, i.e. the order in which the monitoring program data move between the system parts 102, 104, 106, 110, can be determined on the basis of the available wireless connection 108, 112, 120. This being so, the update may first be performed between the parts 102, 104, 106, 110 in the connection, the part 102, 104, 106, 110 without a connection can be updated when a wireless connection 234 is available. Accordingly, for example, monitoring program data to be input with the computer 110 is first updated in the terminal 106, which is usually carried along by the user at all times. The performance monitor 102, 104 receives a monitoring program data update from the terminal 106 when the distance between the performance monitor 102, 104 and the terminal 106 is sufficiently short for establishing the wireless connection 234.

In another situation, the performance monitor 102, 104 may update the terminal 106 with the measurement data immediately after an exercise. This being so, the user 100 may transfer the measurement data to the computer 110 by means of the terminal 106 by using the update according to the solution presented.

In an embodiment, the application executes the monitoring program data output process. In this case, the monitoring program data can be output to the user's 100 usage from the performance monitor 102, 104, from the mobile communications system terminal 106 and from the computer 110. For example, the user 100 may check the entries in the program calendar by using the performance monitor 102, 104, the mobile communications system terminal 106 and the computer 110. This being so, the monitoring program data have been updated before the application is used.

In an embodiment, the monitoring program data are checked in the performance monitor 102, 104, the terminal 106 and the computer 110. If the monitoring program data are less than predetermined conditions, a command for updating the performance monitor 102, 104, the terminal 106 and the computer 110 with the updateable monitoring program data is generated. The system may comprise an update protocol that compares the latest update time of the monitoring data elements located in the different system parts 102, 104, 106, 110 and/or the contents of the monitoring data elements, for example, and update the monitoring program data elements with the updateable elements.

In an embodiment, the system checks the monitoring program data automatically in the performance monitor 102, 104, the terminal 106 and the computer 110. The check may be initiated for instance when the system observes that the wireless connection 108, 112, 120 is operable. However, the system may request verification from the user to initiate the update.

In an embodiment, the check of the monitoring program data is timed once a predetermined monitoring program data time term is fulfilled. The user 100 may be able to set the predetermined time term and it may generate monitoring program data elements. For example, the system may perform the check at a given point of time, which may a given weekday. If the wireless connection 108, 112, 120 is not operable at said point in time, the check may be executed when the wireless connection 108, 112, 120 is operable the following time. The predetermined time term may also be determined during the time that has passed from the previous update.

In an embodiment, the application performs a physiological state-monitoring program calculation algorithm by processing the monitoring program data. In this case, the performance monitor 102, 104, the mobile communications system terminal 106 and the computer 110 are each separately able to execute the calculation algorithm. For example, the calculation algorithm may calculate for instance a target variable, such as the user's 100 target weight, the user's 100 energy intake target, the user's 100 energy consumption target or another variable requiring processing. The operation of calculation algorithms in a performance monitor is known *per se.* The monitoring program data update may be performed before the calculation algorithm is performed. If the calculation algorithm generates new monitoring program data, the other system parts may be updated with the updateable monitoring program data after the calculation algorithm is performed.

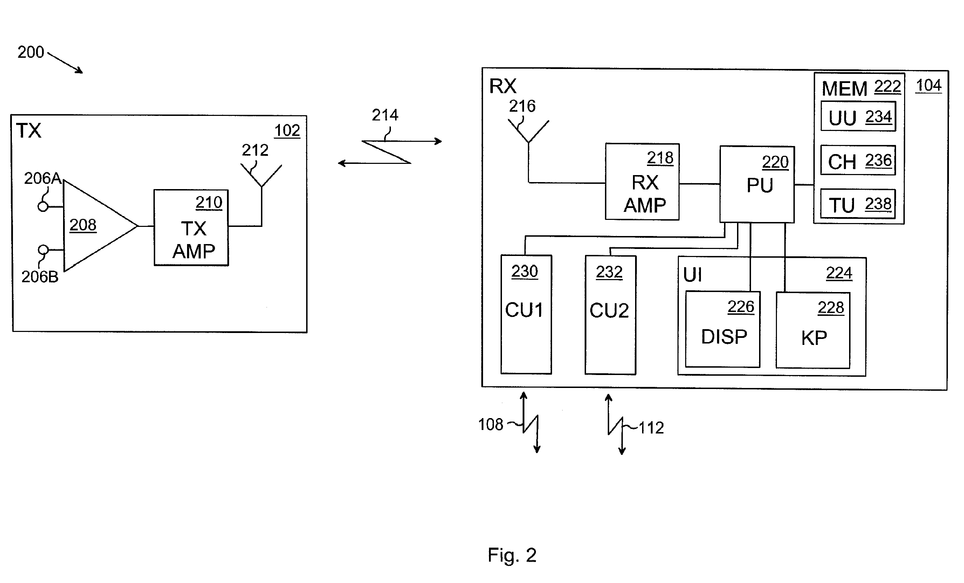

Figure 2 shows an example of the structure of a performance monitor 200 based on wireless data transfer. In the example of Figure 2, the measurement is directed at a user's electrocardiogram, but the solution presented is not restricted to information obtained from an electrocardiogram.

The performance monitor 200 typically comprises electrodes 206A, 206B, an ECG preamplifier 208 (ECG, Electrocardiogram) provided with differential feed poles, a transmitter amplifier (TX AMP) 210, a transmitter antenna 212, a receiver antenna 216, a receiver amplifier (RX AMP) 218, a processing unit (PU) 220, a memory unit (MEM) 222, and a user interface (UI) 224.

The electrodes 206A, 206B experience the electrical potential generated by the electric activity of the myocardium and generate an ECG signal characteristic of the electric activity of the myocardium. The ECG signal is supplied from the electrodes 206A, 206B to the ECG preamplifier 208.

The ECG preamplifier 208 preamplifies the ECG signal and supplies the preamplified ECG signal to the transmitter amplifier 210. The transmitter amplifier 210 may comprise a plurality of successive amplifier stages, such as AGC (Automatic Gain Control), an amplifier and a power amplifier.

The amplified ECG signal is supplied to the transmitter antenna 212, which generates an electromagnetic field 214 for transferring ECG data. The ECG data may comprise for instance the ECG as such, part of the ECG and/or timing information about the heart rate. The timing information may comprise a timing pulse representative of the timing of a predetermined part of the ECG.

The timing information may be determined for instance by identifying the QRS complex of the ECG and by determining the timing of the QRS complex. The QRS complex may be expressed with a pulse detector, for example. For example, the transmitter part 102 may generate a burst that represents the timing of each pulse and that is transmitted to the receiver part 104. The receiver part 104 receives the bursts and may determine the pulse interval between the successive bursts, for example.

In the example presented, the magnetic component of the electromagnetic field 214 generates a wireless connection. However, the solution is not restricted to the use of the magnetic component of the electromagnetic field 214, but any wireless data transmission form may be employed in the transfer of the ECG data.

The receiver antenna 216 detects the electromagnetic field 214 generated by the transmitter antenna 212 and generates an induced electric signal, which is supplied to the receiver amplifier 218.

The receiver amplifier 218 performs processing of the electric signal, such as filtering and amplification. In addition, the receiver amplifier may comprise a plurality of successive regulation stages.

The receiver amplifier 218 supplies the electric signal to the processing unit 220, which may perform analog signal modification of the electric signal, such as filtering and analog-to-digital conversions. In addition, the processing unit 220 may perform digital processing, such as digital filtering, signal modification, detection of the ECG signal, and analysis of the ECG signal.

The processing unit 220 may determine the value of the pulse variable characteristic of the pulse. The pulse variable may be pulse interval, pulse frequency, variation in pulse interval and/or variation in pulse frequency.

The processing unit 220 also executes applications of the physiological data-monitoring program.

The processing unit 220 may be implemented by the use of analog circuits, ASIC circuits (Application Specific Integrated Circuit), a digital processor, memory and computer software. The processing unit 220 may constitute part of the computer of the performance monitor 200.

Part of the data produced by the processing unit 220 and the monitoring program data can be stored in a memory unit 222 connected to the processing unit 220. In addition, the memory unit 222 may comprise coded instructions for executing a computer process in the processing unit 220.

The user interface 224 typically comprises a display unit 226 and a display controller. The display unit 226 may comprise LCD (Liquid Crystal Display) components, for example.

The user interface 224 further comprises a keypad 228 by means of which a user can input commands in the performance monitor 200.

The memory unit 222 may comprise coded instructions for executing an application that executes at least part of the physiological state-monitoring program by processing the monitoring program data of the physiological state-monitoring program.

In addition, the memory unit 222 may comprise coded instructions 234 for updating the performance monitor 200, the terminal 106 and the computer 110 with updateable monitoring program data. This being so, the coded instructions comprise for instance instructions for executing an update protocol between the performance monitor 200 and the terminal 106, and coded instructions for executing an update protocol between the performance monitor 200 and the computer.

The memory unit 222 may also comprise coded instructions 236 for checking the monitoring program data in the performance monitor 200, the terminal 106 and the computer 110.

The memory unit 222 may further comprise coded instructions 234 for updating the performance monitor 200, the terminal 106 and the computer 110 with updateable monitoring program data in case the monitoring program data are less than predetermined conditions.

In addition, the memory unit 222 may comprise coded instructions 238 for initiating the check of the monitoring program data once a predetermine time condition is fulfilled.

The memory unit 222 may also comprise coded instructions for executing an application that executes an input process for the monitoring program data. In this case, the user 100 may use the keypad 228 to input monitoring program data in the performance monitor 200.

The memory unit 222 may further comprise coded instructions for executing an application that executes an output process for the monitoring program data. In this case, the output can be directed to the display unit 226, which may display the monitoring program data numerically and/or graphically.

The transmitter part 102 shown in Figure 2 typically comprises device parts 206A to 212 and performs ECG measurement and the transmission of ECG data to the receiver part 104. In some embodiments, the transmitter part 102 may comprise a heart rate detector that detects a predetermined part from the ECG, generates a transmitter burst and/or a bit stream descriptive of the timing of the predetermined part of the ECG, and transmits the transmitter burst to the receiver part 104.

The receiver part 104 typically comprises device parts 216 to 128, which process the electric signal employed in wireless data transfer and ECG data, and produce a user interface.

Referring to the embodiment presented in Figure 1, the transmitter part 102 is used around the user's 100 thorax. ECG information is transferred wirelessly from the transmitter part 102 to the receiver part 104, which is typically a wrist device disposed in the wrist. In some embodiments, the receiver part 104 is attachable to bicycle structures, such as the handlebar. However, the location of the receiver part 104 is not restricted to the wrist or the handlebar, but is can be placed wherever with the proviso that the wireless connection between the transmitter part 102 and the receiver part 104 is maintained.

In an embodiment, the transmitter part 102 and the receiver part 104 are integrated into the same performance monitor, whereby a performance monitor to be kept in the wrist or the bicycle handlebar, for example, is created. In this case, some of the device parts, such as antennas 212, 216 and amplifiers 210, 218, shown in Figure 2, may not be required. In an embodiment, the transmitter part 102 and part of the receiver part 104 are integrated into a transmitter belt 102, whereby the transmitter belt 102 is able to collect ECG data, process ECG data and determine values of variables characteristic of the heart rate. This being so, the wireless connection transfers processed data, such as values of variables characteristics of the heart rate and commands issued by the user, from the transmitter belt 102 to the receiver part 104. The receiver part 104 may be also wirelessly, optically or galvanically connected to the transmitter belt 102.

In an embodiment, the performance monitor 200 of Figure 2 comprises an acceleration sensor that can be connected to the processing unit 220. The signals generated by the acceleration sensor can be used to conclude the user's sporting activity. The activity can be used to determine the amount of energy consumed by the user, for example. In an embodiment, activity measurement replaces heart rate measurement, whereby the performance monitor 200 does not comprise elements performing heart rate measurement. The acceleration sensor can be placed in the receiver part 104, whereby no transmitter part 102 is necessarily required.

Figure 2 also shows a first communication unit 230 and a second communication unit 232. The first communication unit 230 implements a wireless connection 108 between the performance monitor 200 and the terminal 106. The second communication unit 232 implements a wireless connection 112 between the performance monitor 200 and the computer 110.

The first communication unit 230 communicates monitoring program data between the performance monitor 200 and the terminal 106.

The second communication unit 232 communicates monitoring program data between the performance monitor 200 and the computer 110.

In an embodiment, the first communication unit 230 and/or the second communication unit 232 is an infrared transceiver. The infrared transceiver can support a standardized protocol, such as IrDA (Infrared Data Association).

In an embodiment, the first communication unit 230 and/or the second communication unit 232 is a radio transceiver, such as a Bluetooth receiver.

In an embodiment, the first communication unit 230 and/or the second communication unit 232 is a voice signal transceiver.

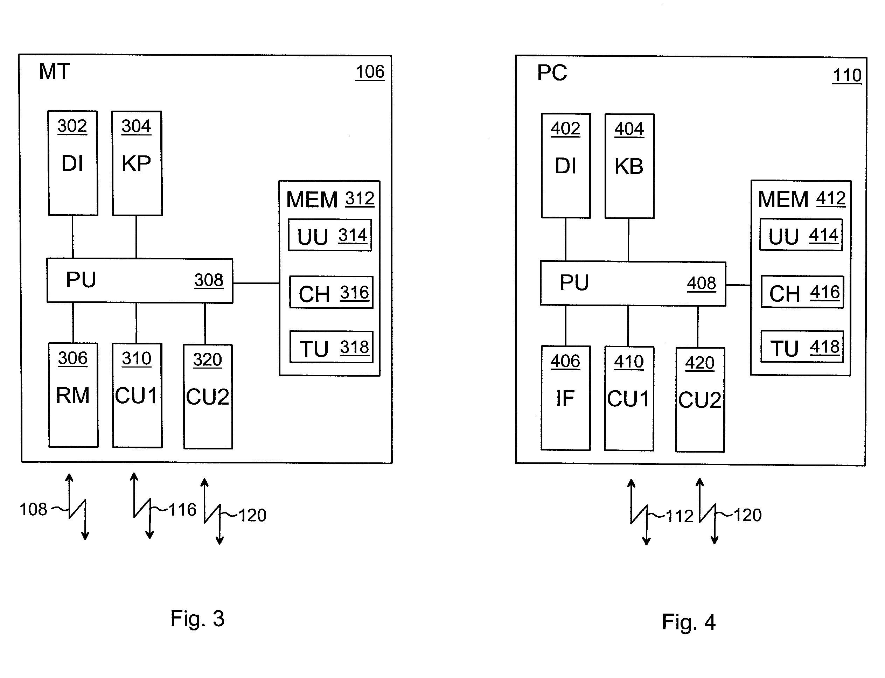

Referring to the example of Figure 3, the terminal 106 comprises a display unit 302, a keypad 304, a radio modem 306, a processing unit 308, a first communication unit 310, a second communication unit 320 and a memory unit 312.

The display unit 302 and the keypad 304 are included in the user interface of the terminal 106, by means of which the user 100 may input and/or observe monitoring program data in some embodiments.

The radio modem 306 comprises a transceiver by means of which the terminal 106 can communicate with the infrastructure 118 of the mobile communications system. Some functionalities of the physiological state-monitoring program can be executed in a server of the public data network 114. In this case, monitoring program data can be communicated between the terminal 106 and the server via the infrastructure 118 of the mobile communications system.

The processing unit 308 comprises a digital processor for executing coded instructions.

In an embodiment, the memory unit 312 comprises coded instructions for executing an application, which executes at least part of the physiological state-monitoring program by processing the monitoring program data of the physiological state-monitoring program.

In an embodiment, the memory unit 312 comprises coded instructions 314 for updating the performance monitor 200, the terminal 106 and the computer 110 with updateable monitoring program data. In this case, the coded instructions comprise coded instructions for executing an update protocol between the performance monitor 200 and the terminal 106, for example.

In an embodiment, the memory unit 312 comprises coded instructions 316 for checking the monitoring program data in the performance monitor 200, the terminal 106 and the computer 110.

In an embodiment, the memory unit 312 comprises coded instructions 314 for updating the performance monitor 200, the terminal 106 and the computer 110 with updateable monitoring program data if the monitoring program data are less than predetermined conditions.

In an embodiment, the memory unit 312 comprises coded instructions 318 for initiating a check of the monitoring program data when a predetermined time condition is fulfilled.

In an embodiment, the memory unit 312 comprises coded instructions for executing an application, which executes an input process of the monitoring program data. In this case, the user 100 can use the keypad 304 to input monitoring program data in the terminal 106.

In an embodiment, the memory unit 312 comprises coded instructions for executing an application, which executes an output process of the monitoring program data. In this case, the output can be directed to the display unit 302, which may display the monitoring program data numerically and/or graphically.

The first communication unit 310 implements a wireless connection between the terminal 106 and the performance monitor 200.

The second communication unit implements a wireless connection 120 between the terminal 106 and the computer 110.

In an embodiment, the first communication unit 310 is an infrared transceiver. The infrared transceiver may support a standardized protocol, such as IrDA.

In an embodiment, the first communication unit 310 is a radio transceiver, such as a Bluetooth receiver.

In an embodiment, the first communication unit 310 is a voice signal transceiver.

In an embodiment, the second communication unit 320 is an infrared transceiver. The infrared transceiver may support a standardized protocol, such as IrDA.

In an embodiment, the second communication unit 320 is a radio transceiver, such as a Bluetooth receiver.

Referring to the example of Figure 4, the computer 110 comprises a display unit 402, a keyboard 404, a network adapter 406, a processing unit 408, a first communication unit 410, a second communication unit 420 and a memory unit 412.

The display unit 402 and the keypad 404 are included in the user interface of the terminal, by means of which the user 100 may input and/or observe the monitoring program data in some embodiments.

The processing unit 408 comprises a digital processor for executing coded instructions.

In an embodiment, the memory unit 412 comprises coded instructions for executing an application that executes at least part of the physiological state-monitoring program by processing the monitoring program data of the physiological state-monitoring program.

In an embodiment, the memory unit 412 comprises coded instructions 414 for updating the performance monitor 200, the terminal 106 and the computer 110 with updateable monitoring program data. This being so, the coded instructions comprise for instance instructions for executing an update protocol between the performance monitor 200 and the computer 110.

In an embodiment, the memory unit 412 comprises coded instructions 416 for checking the monitoring program data in the performance monitor 200, the terminal 106 and the computer 110.

In an embodiment, the memory unit 412 comprises coded instructions 414 for updating the performance monitor 200, the terminal 106 and the computer 110 with updateable monitoring program data in case the monitoring program data are less than predetermined conditions.

In an embodiment, the memory unit 412 comprises coded instructions 418 for initiating the check of the monitoring program data once a predetermine time condition is fulfilled.

In an embodiment, the memory unit 412 comprises coded instructions for executing an application that performs an input process for the monitoring program data. In this case, the user 100 may use the keyboard 404 to input monitoring program data in the computer.

In an embodiment, the memory unit 412 comprises coded instructions for executing an application that performs an output process for the monitoring program data. In this case, the output can be directed to the display unit 402, which may display the monitoring program data numerically and/or graphically.

The communication unit 410 implements a wireless connection 108 between the computer 110 and the performance monitor 200.

In an embodiment, the communication unit 410 is an infrared transceiver. The infrared transceiver may support a standardized protocol, such as the IrDA.

In an embodiment, the communication unit 410 is a radio transceiver, such as a Bluetooth receiver.

In an embodiment, the communication unit 410 is a voice signal transceiver.

In an embodiment, the second communication unit 420 is an infrared transceiver. The infrared transceiver may support a standardized protocol, such as the IrDA.

In an embodiment, the second communication unit 420 is a radio transceiver, such as a Bluetooth receiver.

A network adapter 406 implements a data connection between the computer 110 and the public data network 114. The data connection may be implemented as wired or wireless. A wireless connection may be implemented via the infrastructure 118 of the mobile communications system and/or by means of a local area network, such as WLAN (Wireless Local Access Network).

As an aspect of the solution presented there is provided a computer software product including coded instructions for executing a computer process in a system. The computer software product can be stored in a distribution medium of a computer program. The distribution medium can be any known medium for distributing a computer program from a manufacturer/vendor to an end user. The distribution medium may for instance a medium readable with a data processing device, a program storage medium or a storage medium, a memory readable with a data processing device or a software distribution package, and a signal understood by a data processing device, a telecommunications signal or a compressed software package.

Although the invention is described herein with reference to the example in accordance with the accompanying drawings, it will be appreciated that the invention is not to be so limited, but it may be modified in a variety of ways within the scope of the appended claims.

## Claims

1. A system for monitoring an individual's physiological state by means of a physiological state-monitoring program, comprising a user-specific performance monitor (102, 104) to be connected wirelessly to the system for generating physiological measurement data about the user, the physiological measurement data being used in the physiological state-monitoring program, **characterized in that**
the user-specific performance monitor (102, 104) comprises processing means (220) for executing an application, the application executing at least part of the physiological state-monitoring program by processing monitoring program data of the physiological state-monitoring program, the system further comprising:
a mobile communications system terminal (106) to be connected wirelessly to the system and configured to execute said application;
a personal computer (110) to be connected wirelessly to the system and configured to execute said application; and
update means (234, 314, 414) for updating the user-specific performance monitor (102, 104), the mobile communications system terminal (106) and the personal computer (110) with updateable monitoring program data by using wireless data transfer.

2. A system as claimed in claim 1, c h a r a c t e r i z e d in that the system comprises first wireless communication means (230, 310) for wireless transfer of the monitoring program data between the user-specific performance monitor (102, 104) and the mobile communications system terminal (106).

3. A system as claimed in claim 1, c h a r a c t e r i z e d in that the system comprises second wireless communication means (232, 410) for wireless transfer of the monitoring program data between the user-specific performance monitor (102, 104) and the personal computer (110).

4. A system as claimed in claim 1, c h a r a c t e r i z e d in that the system comprises third wireless communication means (320, 420) for wireless transfer of the monitoring program data between the mobile communications system terminal (106) and the personal computer (110).

5. A system as claimed in claim 1, c h a r a c t e r i z e d in that the system further comprises checking means (236, 316, 416) for checking the monitoring program data in the user-specific performance monitor (102, 104), the mobile communications system terminal (106) and the personal computer (110), the checking means (236, 316, 416) being configured to generate a command to update the user-specific performance monitor (102, 104), the mobile communications system terminal (106) and the personal computer (110) with updateable monitoring program data if the monitoring program data are less than predetermined conditions.

6. A system as claimed in claim 5, c h a r a c t e r i z e d in that the system further comprises timing means (238, 318, 418) for initiating the check on the monitoring program data when a predetermined time condition is fulfilled.

7. A system as claimed in claim 1, c h a r a c t e r i z e d in that an application executes an input process for the monitoring program data.

8. A system as claimed in claim 1, c h a r a c t e r i z e d in that an application executes an output process for the monitoring program data.

9. A system as claimed in claim 1, c h a r a c t e r i z e d in that an application executes a calculation algorithm for the physiological state-monitoring program by processing the monitoring program data.

10. A computer software product including coded instructions for executing a computer process in a system for monitoring an individual's physiological state by means of a physiological state-monitoring program, the system comprising a user-specific performance monitor to be connected wirelessly to the system for generating physiological measurement data about the user, the physiological measurement data being used in the physiological state-monitoring program, **characterized in that** the system further comprises a mobile communications system terminal to be connected wirelessly to the system and a personal computer to be connected wirelessly to the system, and that the computer software program comprises:
coded instructions for executing an application in a measuring unit, in the mobile communications system terminal and in the personal computer, the application executing at least part of the physiological state-monitoring program by processing the monitoring program data of the physiological state-monitoring program; and
coded instructions for updating the user-specific performance monitor, the mobile communications system terminal and the personal computer with updateable monitoring program data by using wireless data transfer.

11. A computer software product as claimed in claim 10, **c h a r -** a c t e r i z e d in that the computer software product further comprises:
coded instructions for checking the monitoring program data in the user-specific performance monitor, the mobile communications system terminal and the personal computer; and
coded instructions for updating the user-specific performance monitor, the mobile communications system terminal and the personal computer with updateable monitoring program data if the monitoring program data are less than predetermined conditions.

12. A computer software product as claimed in claim 11, **c h a r -** a c t e r i z e d in that the computer software product further comprises coded instructions for initiating a check on the monitoring program data when a predetermined time condition is fulfilled.

13. A computer software product as claimed in claim 10, c h a r - a c t e r i z e d in that the computer software product further comprises coded instructions for wirelessly transferring the monitoring program data between the user-specific performance monitor and the mobile communications system terminal.

14. A computer software product as claimed in claim 10, **c h a r -** a c t e r i z e d in that the computer software product further comprises coded instructions for wirelessly transferring the monitoring program data between the user-specific performance monitor and the personal computer.

15. A computer software product as claimed in claim 10, **c h a r -** a c t e r i z e d in that the computer software product further comprises coded instructions for wirelessly transferring the monitoring program data between the mobile communications system terminal and the personal computer.

16. A computer software product as claimed in claim 10, **c h a r -** a c t e r i z e d in that an application executes an input process for the monitoring program data.

17. A computer software product as claimed in claim 10, c h a r - a c t e r i z e d in that an application executes an output process for the monitoring program data.

18. A computer software product as claimed in claim 10, c h a r - a c t e r i z e d in that an application executes a calculation algorithm for the physiological state-monitoring program by processing the monitoring program data.

19. A user-specific performance monitor for generating physiological measurement data about a user, the user-specific performance monitor being wirelessly connectable to a system, the system being used to monitor an individual's physiological state by means of a physiological state-monitoring program, the physiological state-monitoring program using physiological measurement data,**characterized in that**
the user-specific performance monitor (102, 104) comprises:
processing means (220) for executing an application, the application executing at least part of the physiological state-monitoring program by processing monitoring program data of the physiological state-monitoring program, the application further being executable in a mobile communications system terminal to be connected wirelessly to the system and in a personal computer to be connected wirelessly to the system; and
update means (234) for updating the system with updateable monitoring program data by using wireless data transfer.
